# EUROPEAN PATENT SPECIFICATION

(11) **EP 2 250 980 B1**
(45) Date of publication and mention of the grant of the patent: **03.08.2011**
(21) Application number: 10158129.6
(22) Date of filing: 29.03.2010
(51) Int. Cl.: A61F 9/00, A61J 1/06

(54) **Kit for customised evaluation and selection of artificial tears**
Kit zur angepassten Bewertung und Auswahl von künstlichen Tränen
Kit pour l'évaluation personnalisée et la sélection de larmes artificielles

(30) Priority: 15.05.2009 IT MI20090855
(43) Date of publication of application: 17.11.2010
(73) Proprietor: Laboratoires THEA, 63100 Clermont-Ferrand (FR)
(72) Inventor: Bertrand, Jean Christophe, 20156, MILANO (IT)
(74) Representative: Denjean, Eric

(56) References cited:
- EP-A1- 0 150 751
- WO-A1-2009/049304
- US-B1- 6 792 939

## Description

The present invention relates to a kit for customised evaluation and selection of artificial tears.

### Prior art

Dry eye syndrome is an abnormality of the tear film which leads to dryness of the eyes. This disorder is dangerous because the tear film tends to break, thus exposing the epithelium to evaporation.

Dry eye syndrome, which is fairly common in elderly people, may be due not only to age, but also to factors such as a reduction in the aqueous component of the tear film, treatment with drugs such as beta-blockers, antihistamines and antidepressants, excessive or improper use of contact lenses.

Dry eye has been treated for some time with artificial tears, namely ophthalmic solutions containing viscosity-controlling polymers which restore the tear film and lubricate the eye, and/or saline and/or sugar solutions.

Examples of polymers conventionally used in the formulation of artificial tears are cellulose derivatives such as hydroxypropyl methylcellulose, hydroxyethylcellulose, guar gum, carboxyvinyl polymers, carbomer, polyvinyl alcohol, polyvinylpyrrolidone, hyaluronic acids and salts thereof, chondroitin sulphate, and dextrans. These polymers of natural, synthetic or semisynthetic origin are often used mixed together, possibly combined with mineral salts, buffers or preservatives.

Specific examples of artificial tear formulations are disclosed in EP 0323522, US 5,770,628, JP 2002255829, US 20050152951, US 20040137079, US 20050260280, US 20050266089, US 20060029571, WO 91/12808 and WO 2003/011305.

EP0150751 discloses a kit comprising a plurality of containers, each one containing a formulation of artificial tears.

The viscosity and lubricating properties of artificial tear formulations must vary within fairly wide limits, so as to meet the differing requirements of a non-uniform population of patients requiring treatment with artificial tears. A formulation which is excessively viscous and/or insufficiently lubricant, and therefore ineffective or unpleasant to use for some patients, may be perfectly suitable for others.

In view of the variety of products available, it is generally possible to meet a very wide range of requirements. However, the problem remains of choosing the formulation which best suits the individual characteristics of a given patient. It is often necessary to buy and test several commercial packs of artificial tears before identifying the one best suited to the characteristics of the eye conditions of a given patient, thus wasting time and money before the correct formulation is identified.

Moreover, patients may merely use the first formulation recommended by their doctor or pharmacist, without trying others which might be better suited to their specific condition.

The aim of the present invention is therefore to provide a kit as disclosed in claim 1 which allows easy evaluation and personalised selection of the artificial tears to be used by a patient requiring artificial tear treatment. The kit according to the invention enables the patient to try and compare a plurality of different formulations in a short time at minimal expense, so as to provide the ophthalmologist with useful information to select the most suitable formulation.

### Description of the invention

It has now been found that the purpose of the invention can be achieved with a kit comprising a plurality of differently marked containers, each of which contains a different artificial tear formulation, suitably labelled so that the user can instil the different formulations sequentially in a suitable time interval, and compare their efficacy so as to continue the treatment with the most effective formulation, which will be purchased separately in the packs and quantities considered appropriate.

In this way, the subjective method of evaluating artificial tears is optimised in terms of reliability (because the patient can compare different formulations under uniform conditions), timing and cost.

The kit will comprise two to ten formulations, each in a separate container, preferably three to six formulations, in particular five formulations, a number which generally allows optimum coverage of the range of viscosity characteristics and lubricating power. The formulations can vary in terms of number and concentration of ingredients.

In a preferred embodiment, the kit according to the invention comprises conventional containers, such as plastic containers, connected to one another by removable elements to form a strip.

Each container is marked with a different number or graphic symbol, to allow convenient, certain identification of each formulation. The containers can be appropriately labelled with the characteristics of the compositions, the methods of application and use, etc.

The kit could also appropriately contain, in addition to the instructions for use, means designed to facilitate the selection of the most effective and best tolerated formulation, and recording of the results obtained with each formulation tested.

For example, a cardboard or plasticised surface could be supplied for this purpose, with the containers or strip of containers removably attached to one side thereof and a number of arbitrary evaluation scales corresponding to the number of formulations in the kit appearing on the other. One example of such scales is known as VAS (Visual Analogue Scale), which relates, for example, to the convenience and pleasantness of use and relief obtainable from each formulation.

The invention will be now be described in greater detail by means of the annexed figure, wherein:
- reference numbers 1 to 5 indicate the containers, each of which contains a different artificial tear formulation,
- numbers 6-10 indicate the markings which identify each container,
- number 11 identifies the connecting elements between the various containers.

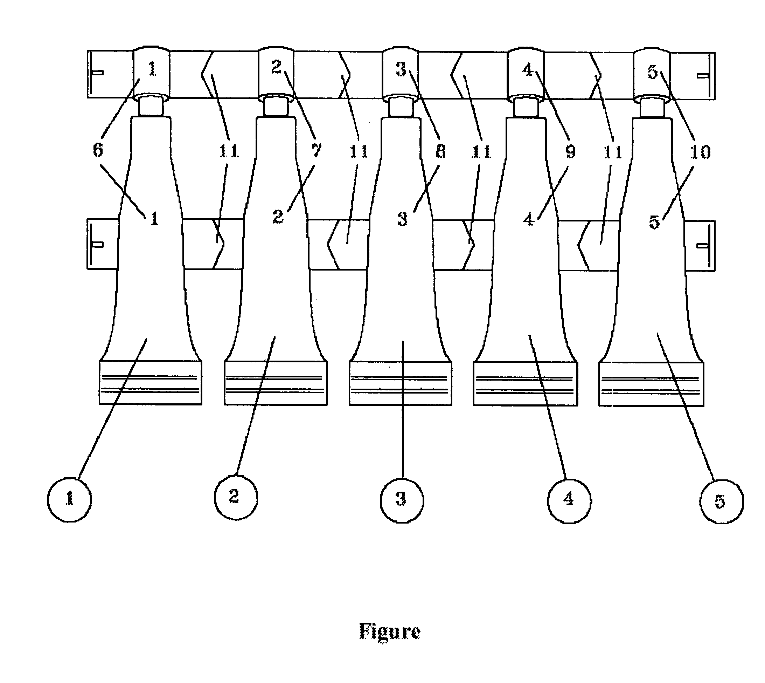

## Claims

1. A kit for the customised evaluation and selection of artificial tears, comprising a plurality of differently marked containers (1-5), each containing a different artificial tear formulation, instructions for use and selection of the most suitable composition, and optionally means bearing a scale designed to evaluate the effectiveness of each composition.

2. Kit as claimed in claim 1, comprising two to ten artificial tear formulations, each in a separate container.

3. Kit as claimed in claim 2, comprising three to six formulations.

4. Kit as claimed in claim 3, containing five formulations.

5. Kit as claimed in any one of claims 1 to 4, in which the containers are connected to each other by removable elements (11) to form a strip.

6. Kit as claimed in any one of claims 1 to 5, in which each container is marked with a different number or graphic symbol (6-10).

## Patentansprüche

1. Set zur kundenspezifischen Bewertung und Auswahl von künstlichen Tränen, mehrere unterschiedlich markierte Behälter (1 - 5) umfassend, wovon jeder eine andere Formulierung künstlicher Tränen, Anweisungen zum Gebrauch und zur Auswahl der geeignetsten Zusammensetzung und optional Einrichtungen enthält, die eine Skala tragen, die dazu ausgelegt ist, die Wirksamkeit jeder Zusammensetzung zu bewerten.

2. Set nach Anspruch 1, zwei bis zehn Formulierungen künstlicher Tränen jeweils in einem separaten Behälter umfassend.

3. Set nach Anspruch 2, drei bis sechs Formulierungen umfassend.

4. Set nach Anspruch 3, fünf Formulierungen enthaltend.

5. Set nach einem der Ansprüche 1 bis 4, wobei die Behälter durch entfernbare Elemente (11) miteinander verbunden sind, um einen Streifen zu bilden.

6. Set nach einem der Ansprüche 1 bis 5, wobei jeder Behälter mit einer anderen Nummer oder einem anderen grafischen Symbol (6 - 10) markiert ist.

## Revendications

1. Kit pour l'évaluation personnalisée et la sélection de larmes artificielles, comprenant une pluralité de récipients marqués de manière différentielle (1-5), chacun contenant une formulation différente de larmes artificielles, des instructions pour l'utilisation et la sélection de la composition la mieux adaptée, et éventuellement des moyens comportant une échelle destinée à évaluer l'efficacité de chaque composition.

2. Kit selon la revendication 1, comprenant de deux à dix formulations de larmes artificielles, chacune dans un récipient séparé.

3. Kit selon la revendication 2, comprenant de trois à six formulations

4. Kit selon la revendication 3, contenant cinq formulations.

5. Kit selon l'une des revendications 1 à 4, dans laquelle les récipients sont connectés les uns aux autres par des éléments détachables (11) pour former un strip.

6. Kit selon l'une des revendications 1 à 5, dans lequel chaque conteneur est marqué avec un numéro différent ou un symbole graphique (6 à 10).
